(19) Europäisches Patentamt
European Patent Office
Office européen des brevets

(11) EP 4 528 751 A1

(12) **EUROPEAN PATENT APPLICATION**
published in accordance with Art. 153(4) EPC

(43) Date of publication:
**26.03.2025 Bulletin 2025/13**

(21) Application number: **23816390.1**

(22) Date of filing: **01.06.2023**

(51) International Patent Classification (IPC):
***G16H 50/20** (2018.01)* ***G16H 50/30** (2018.01)*
***G16H 30/40** (2018.01)* ***G06N 3/04** (2023.01)*
***G06V 10/70** (2022.01)* ***G06V 20/69** (2022.01)*

(52) Cooperative Patent Classification (CPC):
**G06N 3/04; G06V 10/70; G06V 20/69; G16H 30/40; G16H 50/20; G16H 50/30**

(86) International application number:
**PCT/KR2023/007538**

(87) International publication number:
**WO 2023/234730 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.06.2022 KR 20220067465**

(71) Applicant: **Deep Bio Inc.**
**Seoul 08380 (KR)**

(72) Inventors:
- **KWAK, Tae Yeong**
  **Seoul 05119 (KR)**
- **LEE, Jae Heon**
  **Siheung-si Gyeonggi-do 14918 (KR)**
- **CHANG, Hye Yoon**
  **Seoul 04153 (KR)**
- **KIM, Sun Woo**
  **Seongnam-si, Gyeonggi-do 13599 (KR)**

(74) Representative: **Flügel Preissner Schober Seidel Patentanwälte PartG mbB**
**Nymphenburger Straße 20**
**80335 München (DE)**

(54) **PATCH LEVEL SEVERITY DETERMINATION METHOD, SLIDE LEVEL SEVERITY DETERMINATION METHOD, AND COMPUTING SYSTEM FOR PERFORMING SAME**

(57) A patch level severity determination method, a slide level severity determination method, and a computing system for performing same are disclosed. According to one aspect of the present invention, provided is a method performed in a computing system including a deep-learning model pre-trained to provide determination results for partial images when each partial image obtained by dividing a pathology slide image is inputted, the method comprising the steps of: for each of a plurality of partial images obtained by dividing a pathology slide image, determining an effective grade for the partial image on the basis of a determination result for the partial image outputted by the deep-learning model that has received the partial image; and determining a slide level severity rating for the entire pathology slide image on the basis of the effective grade for each of the plurality of partial images that constitute the pathology slide image.

FIG. 7

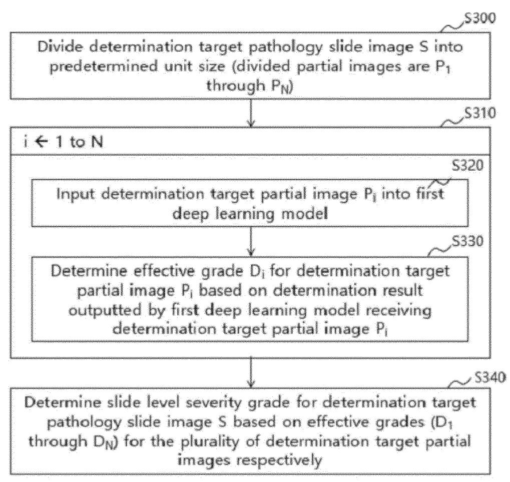


EP 4 528 751 A1

## Description

### Technical Field

The present disclosure relates to a patch level severity determination method, a slide level severity determination method, and a computing system for performing the same, and more specifically, relates to a method for determining an effective grade by utilizing an output of a deep learning model trained to receive a pathology image and output histological severity grade information, a method for determining slide level severity for the entire tissue included in the pathology slide image by utilizing an output of a deep learning model trained to analyze a predetermined partial image including the tissue and determine histological severity of a disease and the computing system for performing the same.

### Background Art

After removing a part or whole of tissues to treat a serious disease such as cancer, histopathological examination is conducted on the excised tissue to predict a patient's prognosis and to determine a treatment method, and severity of the disease is diagnosed.

There are agreed-upon systems for reporting histological severity for each disease, and these systems grade and report the severity based on morphological features of the tissue. For example, for a breast cancer, there is a grading system called the Nottingham system, which grades the severity of the tissue by measuring how well a tissue structure is maintained (tubule formation), how uniform cell nuclei are (nuclear pleomorphism), and how much mitosis occurs (mitotic activity), grading on a scale of 1 through 3 and then adding up. For an ovarian cancer, there is a grading system called the Silverberg system similar to the Nottingham system, which also grades the tissue structure, the cell nucleus, and the mitosis on a scale of 1 through 3 for each category, and then grades the severity based on this. For a prostate cancer, there is a grading system called the Gleason grading system, which classifies tissue patterns according to morphological features of gland tissues on a scale of 1 through 5, and grades the overall severity according to a ratio of each grade area in the entire tissue.

The biggest problem with these grading systems is that it is very difficult to accurately determine the grade of the lesion according to the system's criteria. Since a shape of biological tissues gradually changes depending on a degree of disease progression, and there may be tissues which may be classified into several grades within a single lesion, it is difficult to determine a single grade frequently.

With recent development of deep learning technology, it is possible to develop a deep learning model with accuracy exceeding human cognitive abilities in an image recognition field. Accordingly, disease diagnosis technology using the deep learning is developed and commercialized in a medical field. The whole slide image generated by scanning biological tissue slides using a digital slide scanner is very large, and if analyzing it to diagnose a disease or to diagnose the severity, the whole slide image is generally divided into small partial images (patches or tiles), each is analyzed with a deep learning model, and results are aggregated to yield a diagnostic result for the whole slide.

If recognizing the severity grade of the tissues by analyzing tissue images with the deep learning model, there is a problem that the accuracy of the recognition results of the model for tissues corresponding to a boundary between grades is not high due to ambiguity in the grade classification as described above. By applying a dual class annotation method (Korean Patent No. 10-2162895; System and method for medical diagnosis supporting dual class) which allows ambiguity in determining tissues in the boundary, it is possible to make the analysis result of the deep learning model for tissues with an ambiguous grade fall in the boundary between grades, but it is very difficult to train to strongly support one grade.

Meanwhile, if the analysis result of the deep learning model does not strongly support one grade, the histological severity of the corresponding tissue may be determined to be in the boundary between two grades, and the histological severity for the whole slide is calculated by considering this boundary information, and thus finer severity grading may be possible compared to the existing histological severity reporting system. Nagpal et at. mentions a method of assigning a value corresponding to a boundary to tissues with an ambiguous grade, in their studies on the histological severity determination technique of prostate cancer (Nagpal, K., Foote, D., Liu, Y. et al. Development and validation of a deep learning algorithm for improving Gleason scoring of prostate cancer. npj Digit. Med. 2, 48 (2019). https://doi.org/10.1038/s41746-019-0112-2). However, this study mentions only the likelihood of this method and the example of assigning values corresponding to the boundary based on the degree of grade agreement of a plurality of pathologists, but does not mention how to use a deep learning model to assign a value corresponding to the boundary of the grade to a lesion or how to grade the severity of the entire tissue by incorporating the severity grades finely assigned to each lesion.

Meanwhile, the existing disease-specific histological severity reporting system mainly determines the severity of the entire tissue in the following manner. For example, the Gleason grading system for the prostate cancer determines the overall severity with a sum of top two severity grades based on an area of a region, and more specifically, represents average severity of the entire area as follows.

TABLE 1

| Severity group | Gleason Score | Average | Note |
|---|---|---|---|
| 1 | 3+3=6 or less | 3 or less | |
| 2 | 3+4=7 | 3~3.5 | |
| 3 | 4+3=7 | 3.5~4 | |
| 4 | 3+5=8 | 3~4 | rare case |
| | 4+4=8 | around 4 | |
| | 5+3=8 | 4~5 | rare case |
| 5 | 4+5=9 | 4~4.5 | |
| | 5+4=9 | 4.5~5 | |
| | 5+5=10 | around 5 | |

In addition, for the breast cancer, since average grades are given to categories of the tissue structure, the cell nucleus, and the cell division for an observed area respectively, and added up to assign the overall grade, the severity of the entire tissue is determined in the form of the average severity of the entire area. However, in some cases, if there is a lesion of a higher severity grade, it may be necessary to assign higher severity regardless of its size. Hence, if the histological severity information for each partial image is outputted, there may be a method of using a maximum value or a mean value in the method of integrating it. However, as shown in the 'rare case' of the prostate cancer severity group mentioned above, it may not be sufficient to determine the severity for the whole merely with the general maximum/minimum/average, and a more accurate slide based severity determination method may be developed by utilizing a machine learning (deep learning) model.

Disclosure of the Invention

Technical Goals

A technical object to be achieved by the present disclosure is to provide a slide level severity determination method capable of more finely distinguishing severity of the whole slide image of biological tissues through deep learning technology.

In addition, another object is to provide a method capable of effectively determining an effective grade of a biological tissue image.

Technical Solutions

According to one aspect of the present disclosure, there is provided a slide level severity determination method performed in a computing system including a first deep learning model which is an artificial neural network pre-trained, if each of partial images obtained by dividing a pathology slide image into a predetermined unit size is inputted, to output a determination result for the inputted partial image, including: for each of a plurality of determination target partial images obtained by dividing a predetermined determination target pathology slide image into the unit size, determining an effect grade for the determination target partial image based on a determination result for the determination target partial image outputted by the first deep learning model receiving the determination target partial image; and determining a slide level severity grade for the whole determination target pathology slide image based on the effective grade for each of the plurality of determination target partial images constituting the determination target pathology slide image, the determination result for the partial image outputted by the first deep learning model is a likelihood value for each grade in a predetermined histological severity grading system with respect to a predetermined disease, a predetermined grade score is pre-assigned to each grade in the severity grading system, and the determining of the effective grade for the determination target partial image based on the determination result for the determination target partial image outputted by the first deep learning model receiving the determination target partial image includes determining the effective grade for the determination target partial image based on the likelihood value of each grade in the severity grading system of the determination target partial image and the grade score for each grade in the severity grading system.

In an embodiment, the determining of the effective grade for the determination target partial image based on the likelihood value of each grade in the severity grading system of the determination target partial image and the grade score for each grade in the severity grading system may include determining the effective grade for the determination target pathology image by calculating a weighted average of grade scores for each grade in the severity grading system weighted by the likelihood value of each grade in the severity grading system; determining the effective grade for the determination target pathology image by calculating a weighted average of grade scores for each of of a plurality of higher grades weighted by a likelihood value of each of the plurality of higher grades of high likelihood values among grades in the severity grading system; or determining a grade score of the highest grade with the highest likelihood value among grades in the severity grading system as the effective grade for the determination target pathology image.

According to another aspect of the present disclosure, there is provided a slide level severity determination method performed in a computing system including a first deep learning model which is an artificial neural network pre-trained, if each of partial images obtained by dividing a pathology slide image into a predetermined unit size is inputted, to output a determination result for the inputted partial image, including: for each of a plurality of determination target partial images obtained by dividing a predetermined determination target pathology slide image into the unit size, determining an effect grade for the determination target partial image based on a determination result for the determination target partial image outputted by the first deep learning model receiving the determination target partial image; and determining a slide level severity grade for the whole determination target pathology slide image based on the effective grade for each of the plurality of determination target partial images constituting the determination target pathology slide image, the determination result for the partial image outputted by the first deep learning model includes a determination result for each pixel constituting the partial image, the determination result for each pixel is a likelihood value for each grade in a predetermined histological severity grading system with respect to a predetermined disease, a predetermined grade score is pre-assigned to each grade in the severity grading system, and the determining of the effective grade for the determination target partial image based on the determination result for the determination target partial image outputted by the first deep learning model receiving the determination target partial image includes, for each determination target pixel constituting the determination target partial image, determining the effective grade for the determination target pixel based on the likelihood value of each grade in the severity grading system of the determination target pixel and the grade score for each grade in the severity grading system; and determining the effective grade for the determination target partial image based on the effective grade of each determination target pixel constituting the determination target partial image.

In an embodiment, the determining of the effective grade for the determination target pixel based on the likelihood value of each grade in the severity grading system of the determination target pixel outputted by the first deep learning model and the grade score for each grade in the severity grading system may include determining the effective grade of the determination target pixel by calculating a weighted average of grade scores for each grade in the severity grading system weighted by the likelihood value of each grade in the severity grading system of the determination target pixel; determining the effective grade of the determination target pixel by calculating a weighted average of grade scores of a plurality of higher grades weighted by the likelihood value of each of the plurality of higher grades of high likelihood values among grades in the severity grading system of the determination target pixel; or determining the grade score of the highest grade with the highest likelihood value among the grades in the severity grading system of the determination target pixel as the effective grade of the determination target pixel.

In an embodiment, the determining of the effective grade for the determination target partial image based on the effective grade of each determination target pixel constituting the determination target partial image may include determining the effective grade for the determination target pathology image by calculating a representative value of the effective grade of each determination target pixel constituting the determination target partial image.

In an embodiment, the determining of the effective grade for the determination target partial image by calculating the representative value of the effective grade of each determination target pixel constituting the determination target partial image may include calculating as the representative value any one of a maximum value, a minimum value, an average value, a median value, and an average value within an interquartile range (IQR) of the effective grade of each determination target pixel constituting the determination target partial image.

In an embodiment, the determining of the slide level severity grade for the whole determination target pathology slide image based on the effective grade for each determination target partial image constituting the determination target pathology slide image may include determining the slide level severity grade for the whole determination target pathology slide image by calculating a representative value of the effective grade of each determination target partial image constituting the determination target pathology slide image.

In an embodiment, the determining of the slide level severity grade for the whole determination target pathology slide image by calculating the representative value of the effective grade of each determination target partial image constituting the determination target pathology slide image may include calculating as the representative value any one of a maximum value, a minimum value, an average value, a median value, and an average value within an IQR of the effective grade of each determination target partial image constituting the determination target pathology slide image.

In an embodiment, the computing system may further include a second deep learning model which is an artificial neural network pre-trained to output an output value for determining the severity grade for the pathology slide image if a data set including the effective grade for each of the plurality of partial images obtained by dividing the pathology slide image into the unit size is inputted, and the determining of the severity grade for the determination target pathology slide image based on the effective grade for each of the plurality of determination target partial images constituting the determination target pathology slide image may include determining the severity grade for the determination target pathology slide image based on the output value outputted by the second deep learning model receiving the data set including the effective grade for each of the plurality of determination target partial images constituting the determination target pathology slide image.

In an embodiment, the second deep learning model may be the artificial neural network pre-trained by a second deep learning model training method, the second deep learning model training method may include obtaining a plurality of training pathology slide images; for each of a plurality of training pathology slides, generating a data set corresponding to the training pathology slide image; and training the second deep learning model by inputting each data set corresponding to the plurality of training pathology slide images into the second deep learning model, and the generating of the data set corresponding to the training pathology slide image may include, for each of a plurality of data set generation partial images obtained by dividing the training pathology slide image into the unit size, determining an effective grade for the data set generation partial image based on a determination result outputted by the first deep learning model receiving the data set generation partial image; and generating the data set including the effective grade for each of the data set generation partial images.

According to another aspect of the present disclosure, there is provided a patch level severity determination method performed in a computing system including a first deep learning model which is an artificial neural network pre-trained, if each of partial images obtained by dividing a pathology slide image into a predetermined unit size is inputted, to output a determination result for the inputted partial image, including: inputting a predetermined determination target partial image into the first deep learning model, and obtaining a determination result for the determination target partial image outputted by the first deep learning model, wherein the determination result for the determination target partial image is a likelihood value for each grade in a predetermined severity grading system for a predetermined disease, and a predetermined grade score is pre-assigned to each grade in the severity grading system; and determining an effective grade for the determination target partial image based on the likelihood value of each grade in the severity grading system outputted by the first deep learning model receiving the determination target partial image and a grade score for each grade in the severity grading system.

According to another aspect of the present disclosure, there is provided a patch level severity determination method performed in a computing system including a first deep learning model which is an artificial neural network pre-trained, if each of partial images obtained by dividing a pathology slide image into a predetermined unit size is inputted, to output a determination result for the inputted partial image, including: inputting a predetermined determination target partial image into the first deep learning model, and obtaining a determination result for the determination target partial image outputted by the first deep learning model, wherein the determination result outputted by the first deep learning model for the determination target partial image includes a determination result for each pixel constituting the determination target partial image, the determination result for each pixel is a likelihood value for each grade in a predetermined severity grading system for a predetermined disease, and a predetermined grade score is pre-assigned to each grade in the severity grading system; for each determination target pixel constituting the determination target partial image, determining an effective grade for the determination target pixel based on the likelihood value of each grade in the severity grading system of the determination target pixel and a grade score for each grade in the severity grading system; and determining an effective grade for the determination target pathology image based on the effective grade of each determination target pixel constituting the determination target partial image.

According to another aspect of the present disclosure, a computer program installed on a data processing device and recorded on a non-transitory medium is provided to perform the method described above.

According to another aspect of the present disclosure, there is provided a non-transitory computer-readable recording medium on which a computer program to perform the method described above is recorded.

According to another aspect of the present disclosure, there is provided a computing system including a processor and a memory, the memory stores a computer program and a first deep learning model which is an artificial neural network pre-trained, if each of partial images obtained by dividing a pathology slide image into a predetermined unit size is inputted, to output a determination result for the inputted partial image , and the computer program, when executed by the processor, controls the computing system to perform the method described above.

In an embodiment, the memory may further store a second deep learning model which is an artificial neural network pre-trained to output an output value for determining a severity grade for the pathology slide image if a data set including the effective grade for each of the plurality of partial images obtained by dividing the pathology slide image into the unit size is inputted.

## Advantageous Effects

According to the technical idea of the present disclosure, it is possible to provide a slide level severity determination method capable of more finely classifying severity of the entire tissue (i.e., the whole slide image) by utilizing an output of a deep learning model trained to determine histological severity of a predetermined disease by analyzing a partial image (e.g., a patch or a tile) which is a part of a whole slide image divided into a predetermined unit size.

In addition, it is possible to provide a method capable of effectively determining an effective grade of a partial image itself by utilizing an output of a deep learning model trained to output histological severity grading information of a biological tissue image.

Brief Description of Drawings

A brief description of each drawing is provided to more fully understand the drawings cited in the detailed description of the present disclosure.

FIG. 1 is a diagram schematically illustrating an example of a configuration of a computing system according to an embodiment of the present disclosure.
FIG. 2 is a diagram for illustrating a first deep learning model according to an embodiment of the present disclosure.
FIG. 3 is a flowchart illustrating an example of a patch level severity determination method performed by a computing system according to an embodiment of the present disclosure.
FIG. 4 is a flowchart illustrating a process for a computing system to train a first deep learning model according to an embodiment of the present disclosure.
FIG. 5A and 5B are drawings for illustrating a first deep learning model according to another embodiment of the present disclosure.
FIG. 6 is a flowchart illustrating an example of a patch level severity determination method performed by a computing system according to another embodiment of the present disclosure.
FIG. 7 is a flowchart illustrating an example of a slide level severity determination method performed by a computing system according to an embodiment of the present disclosure.
FIG. 8 is a diagram illustrating a specific example of applying a slide level severity determination method according to an embodiment of the present disclosure.
FIG. 9 is a diagram illustrating a process for a computing system to train a second deep learning model according to an embodiment of the present disclosure.

Best Mode for Carrying Out the Invention

Since the present disclosure may make various modifications and have various embodiments, specific embodiments will be illustrated in the drawings and described in detail in the detailed description. However, this is not intended to limit the present disclosure to specific embodiments, and it should be understood to embrace all changes, equivalents and alternatives included in the spirit and the scope of the present disclosure. **In** describing the present disclosure, if it is determined that a detailed description of related known technologies may obscure the gist of the present disclosure, the detailed description will be omitted.

Terms such as first and second may be used to describe various components, but the components should not be limited by the terms. The above terms are used only for the purpose of distinguishing one component from another component.

Terms used in the present application are used only to describe a particular embodiment, and are not intended to limit the present disclosure. Singular expressions include plural expressions unless the context clearly means otherwise.

**In** this specification, terms such as "include" or "have" are intended to designate presence of a feature, a number, a step, an operation, a component, a part, or a combination thereof described in the specification, and it should be understood that they do not preclude the possibility of the presence or addition of one or more other features, numbers, steps, operations, components, parts, or combinations thereof.

**In** addition, in the present specification, if one component 'transmits' data to another component, it means that the component may directly transmit the data to the other component or may transmit the data to the other component via at least one another component. Conversely, if one component 'directly transmits' data to another component, it means that the data is transmitted from the component to the other component without passing through yet another component.

Hereinafter, the present disclosure will be described in detail based on on embodiments of the present disclosure, with reference to the accompanying drawings. Like reference numerals in each drawing indicate like members.

A patch level severity determination method and a slide level severity determination method according to the technical idea of the present disclosure may be carried out by a computing system. The computing system may be a data processing device with computing capability, and may include a computing device such as a personal computer or a mobile device as well as a server which is a data processing device generally accessible by a client via a network.

FIG. 1 is a diagram schematically illustrating an example of a configuration of a computing system according to an embodiment of the present disclosure. A computing system 100 may physically have a configuration as shown in FIG. 1.

The computing system 100 may be provided with a computer program 123 for implementing the technical idea of the present disclosure, a memory 120 for storing a first deep learning model 121, and a processor 110 for executing the computer program 123 stored in the memory 120. According to an embodiment, the memory 120 may further store a second deep learning model 122.

An average expert in the technical field of the present disclosure may easily infer that the processor 110 may be referred to as various terms such as a central processing unit (CPU), an accelerated processing unit (APU), a microprocessor, or an application specific integrated circuit (ASIC), according to an implementation of the computing system 100. In addition, the computing system 100 may be implemented by organically combining a plurality of physical devices, and in such a case, an average expert in the technical field of the present disclosure may easily infer that the at least one processor 110 is provided for each physical device to implement the computing system 100 of the present disclosure. The processor 110 may further include a graphics processing unit (GPU) used to train the first deep learning model 121 and/or the second deep learning model 122.

The memory 120 stores the the program 123, and may be implemented as any type of a storage device accessible by the processor 110 to run the program 123. In addition, the memory 120 may be implemented with a plurality of storage devices rather than a single storage device according to hardware implementation. In addition, the memory 120 may include a temporary memory device, as well as a main memory device. Further, the memory 120 may be implemented as a volatile memory or a non-volatile memory. The memory 120 may include, for example, a flash memory, a read only memory (ROM), a random access memory (RAM), an electrically erasable ROM (EEROM), an electrically erasable ROM (EPROM), electrically erasable programmable ROM (EEPROM), a hard disk, and a register. Alternatively, the memory 120 may be defined as including any form of information storage means implemented to store the program 123 and to be driven by the processor 110.

The computing system 100 may, according to an embodiment, be further provided with various peripheral devices (a first peripheral through an M-th peripheral 130-1 through 130-M). For example, an average expert in the technical field of the present disclosure may easily infer that the computing system 100 may further include a keyboard, a display device, a graphics card, a networking device, a storage device, and so on as peripherals.

The computing system 100 may be implemented with any one physical device, but an average expert in the technical field of the present disclosure may easily infer that the computing system 100 may be implemented by organically combining a plurality of physical devices as needed according to the technical idea of the present disclosure.

Hereinafter, an average expert in the technical field of the present disclosure may easily infer that, if a specific module performs a specific function, it means that the processor 110 performs the function by running the program 123 provided in the memory 120 in the present specification.

In this specification, a deep learning model includes a multilayer perceptron model, which may indicate a set of information which represents a series of architectures for defining an artificial neural network.

In an embodiment, the deep learning model may be a convolutional neural network. The convolutional neural networks, as is well known, may include an input layer, a plurality of hidden layers, and an output layer. The plurality of hidden layers each may include a convolutional layer and a pooling layer (or a subsampling layer). The convolutional neural network may be defined by a function, a filter, a stride, a weight factor, and so on for defining each of such layers. In addition, the output layer may be defined as a fully connected FeedForward layer.

The architecture of each layer constituting the convolutional neural network is widely known. For example, known functions may be used for the number of layers to be included in the plurality of layers, a convolutional function, a pooling function, and an activation function for defining the plurality of layers respectively, or functions separately defined may be used to implement the technical idea of the present disclosure.

An example of the convolutional function includes a discrete convolutional sum and so on. An example of the pooling function may use max pooling, average pooling, and so on. An example of the activation function may include sigmoid, tangent hyperbolic (tanh), rectified linear unit (ReLU) and the like. If the architecture of the convolutional neural network is defined, the convolutional neural network with the defined architecture may be stored in a storage device. If the convolutional neural network is trained, weight factors corresponding to the layers respectively may be specified. That is, the training of the convolutional neural network may indicate a process of determining the the weight factors of the layers respectively. If the convolutional neural network is trained, the trained convolutional neural network may receive input data through the input layer and output output data through a predefined output layer.

The neural network according to an embodiment of the present disclosure may be defined by selecting one or more of the widely known architectures as mentioned above, or an independent architecture may be defined for the neural network.

The neural network may be a classification neural network which may be used for classification of the inputted data. The classification neural network may be a neural network for classifying a determination result of the inputted data into any one of a plurality of predefined results.

According to an embodiment, the neural network may be a segmentation neural network. The segmentation neural network is a neural network for specifying an area which satisfies a specific condition (e.g., an area of disease outbreak) in the inputted image, and may be also referred to as a pixel level classification neural network which performs pixel-based classification.

Meanwhile, the first deep learning model 121 may be an artificial neural network pre-trained, if a partial image of a pathology slide image divided into a predetermined unit size is inputted, to output a determination result for the inputted partial image. The pathology slide image may be a whole slide image of a pathology specimen scanned with a digital scanner.

The digital slide image of the pathology specimen may be generated by slicing the pathology specimen to make glass slides, then staining it with a predetermined dye and digitizing it. The pathology specimen may be biological tissues removed from various organs of a human body using biopsy and surgical excision.

The partial image inputted into the first deep learning model 121 is a part of the pathology slide image divided into a predetermined unit size, and may be referred to as a patch or a tile.

In an embodiment, the first deep learning model 121 may be a patch level classification neural network. In this case, the first deep learning model 121 may be an artificial neural network pre-trained to output a determination result for an inputted pathology image if a partial image is inputted, the determination result for the pathology image outputted by the first deep learning model is a likelihood value for each grade in a predetermined histological severity grading system for a predetermined disease, and a predetermined grade score may be given to each grade in the severity grading system. For example, the severity grading system may be the Nottingham system, the Silverberg system, the Gleason grading system, and so on.

FIG. 2 is a diagram for illustrating a first deep learning model 121 according to an embodiment of the present disclosure.

Referring to FIG. 2, the first deep learning model 121 may receive a partial image 1 through the input layer, and output a determination result 2 for the inputted partial image 1 through the output layer. The determination result 2 may be, for example, a likelihood value for each grade of prostate cancer severity grades according to the Gleason grading system. In this case, the sum of the likelihood value for each class may be 1.

Meanwhile, a grade score 3 may be pre-assigned for each grade in the Gleason grading system.

FIG. 3 is a flowchart illustrating an example of a patch level severity determination method performed by the computing system 100. The computing system 100 may use the first deep learning model 121 as shown in FIG. 2, to determine the severity (precisely, an effective grade in a predetermined histological severity grading system for a predetermined disease) of the partial image, in other words, the patch level severity.

Referring to FIG. 3, the computing system 100 may input a predetermined determination target partial image into the first deep learning model 121, and obtain a determination result of the determination target partial image outputted by the first deep learning model 121 (S100). At this time, the determination result of the determination target partial image is a likelihood value for each grade in a predetermined severity grading system for a predetermined disease, as mentioned above, and a predetermined grade score may be pre-assigned to each grade in the severity grading system.

Next, the computing system 100 may determine the effective grade for the determination target partial image based on the determination result of the determination target partial image (S110).

In an embodiment, the computing system 100 may determine the effective grade for the determination target partial image by calculating a weighted average of the grade score for each grade in the severity grading system weighted by the likelihood value of each grade in the severity grading system. Taking the case of FIG. 2 as an example, the computing system 100 may input the partial image 1 of FIG. 2 into the first deep learning model 121 to obtain the likelihood value 2 for each Gleason grade, and calculate the weighted average of the grade score for each grade of the Gleason grading system weighted by the likelihood value of each grade in the Gleason grading system. In this case, a benign may be excluded in calculating the weighted average, and the effective grade calculated in the case of FIG. 2 is as follows (rounded to the fourth decimal place).

$$(3\times0.1 + 4\times0.5 + 5\times0.3) / (0.1+0.5+0.3) = 3.8 / 0.9 = 4.222$$

In another embodiment, the computing system 100 may determine the effective grade for the determination target partial image by calculating a weighted average of grade scores for the plurality of higher grades weighted by the likelihood value of each of the higher grades of high likelihood values among grades in the severity grading system. Assuming that the computing system 100 calculates a weighted average for top two likelihood grades weighted by the likelihood values of the grades as the effective grade, the computing system 100 may calculate the effective grade in the case of FIG. 2 as below.

$$(4\times0.5 + 5\times0.3) / (0.5+0.3) = 3.5 / 0.8 = 4.375$$

In another embodiment, the computing system 100 may determine a grade score of the highest grade of the highest likelihood value among the grades in the severity grading system as the effective grade for the determination target partial image, and the computing system 100 may determine the grade score 4 of the grade G4 with the greatest likelihood of 0.5 as the effective grade in the case of FIG. 2.

Meanwhile, the first deep learning model 121 is a pre-trained artificial neural network, and FIG. 4 is a flowchart illustrating a process of training the first deep learning model 121.

Referring to FIG. 4, the computing system 100 may obtain a plurality of training partial images (S200), and at this time, the plurality of training earning partial images each may be tagged with any one of the grades of the histological severity grading system. The plurality of training partial images may be generated by dividing one or more whole training slide images into a unit size.

The computing system 100 may input each of the plurality of training partial images into the first deep learning model 121 to train the first deep learning model 121.

To briefly explain the deep learning process based on training data tagged with a correct answer label, if the deep learning model receiving the training data outputs a result, an error between the output result and the correct answer label may be reflected in the deep learning model through a backpropagation process, a technique such as gradient descent may be applied in the backpropagation process, and the detailed process of training the model from the training data through such a deep learning process is very well known, which shall be omitted in further detailed explanation.

The embodiment of the present disclosure described earlier in reference to FIG. 2 through FIG. 4 relates to the case where the first deep learning model 121 is the classification neural network, that is, the first deep learning model 121 is a neural network for determining which class (grade) the inputted image corresponds to.

However, according to another embodiment of the present disclosure, the first deep learning model 121 may be a segmentation neural network or a pixel level classification neural network, which will be described in more detail by referring to FIG. 5 through FIG. 6.

FIG. 5A and FIG. 5B are diagrams for illustrating a first deep learning model 121-1 which is a segmentation neural network of the present disclosure.

Referring first to FIG. 5A, the first deep learning model 121-1 may receive a partial image 10 through the input layer, and the partial image 10 may include a plurality of pixels (e.g., 11). The example of FIG. 5A illustrates that the partial image 10 includes 4×4 pixels. Meanwhile, the first deep learning model 121-1 may output a determination result 20 for the inputted partial image 10, and the determination result 20 for the partial image 10 may include a determination result (e.g., 21) for each pixel constituting the partial image 10. In the example of FIG. 5A, the determination result 20 for the partial image 10 may include the determination result for each of the 4×4 pixels.

FIG. 5B is the diagram illustrating a determination result of any one of the pixels constituting the partial image 10 of FIG. 5A, and referring to FIG. 5B, the determination result 21 of the pixel 11 may be, for example, a likelihood value of each grade of prostate cancer severity grades according to the Gleason grading system. In this case, the sum of the likelihood values for each grade may be 1. Meanwhile, a grade score 3 may be pre-assigned for each grade in the Gleason grading system.

FIG. 6 is a flowchart illustrating another example of a patch level severity determination method performed by the computing system 100. The computing system 100 may determine severity of a partial image (precisely, an effective grade in a predetermined histological severity grading system for a predetermined disease) by using the first deep learning model 121-1 of FIG. 5A.

Referring to FIG. 6, the computing system 100 may input a predetermined determination target partial image into the first deep learning model 121-1, and obtain a determination result of the determination target partial image outputted by the first deep learning model 121-1 (S150). At this time, the determination result of the determination target partial image may include a determination result of each determination target pixel constituting the determination target partial image as described above, the determination result for each determination target pixel may be a likelihood value for each grade in the predetermined severity grading system for the predetermined disease, and a predetermined grade score may be pre-assigned to each grade in the severity grading system.

Next, for a determination target pixel p constituting the determination target partial image, the computing system 100 may determine an effective grade of the determination target pixel p based on the determination result of the first deep learning model 121-1 (S160) for the determination target pixel p.

There may be various methods for determining the effective grade of the determination target pixel p.

In an embodiment, the computing system 100 may determine the effective grade for the determination target pixel p by calculating a weighted average of grade scores for each grade in the severity grading system weighted by the likelihood value of each grade in the severity grading system. Taking the case of FIG. 5B as an example, the computing system 100 may obtain the likelihood value 21 for each Gleason grade for the pixel 11 of FIG. 5B, and calculate the weighted average of the grade scores for each grade in the Gleason grading system weighted by the likelihood value of each grade in the Gleason grading system. In this case, the benign may be excluded in calculating the weighted average, and the effective grade calculated in the case of FIG. 5B is as follows.

$$(3\times0.1 + 4\times0.3 + 5\times0.4) / (0.1+0.3+0.4) = 3.5 / 0.8 = 4.375$$

In another embodiment, the computing system 100 may determine the effective grade for the determination target pixel p by calculating a weighted average of grade scores of the plurality of higher grades weighted by a likelihood value of each of the higher grades of high likelihood values among the grades in the severity grading system. Assuming that the computing system 100 calculates a weighted average for top two likelihood grades weighted by the likelihood values of the grades as the effective grade, the computing system 100 may calculate the effective grade of the pixel 11 in the case of FIG. 5B as follows (rounded to the fourth decimal place).

$$(4\times0.3 + 5\times0.4) / (0.3+0.4) = 3.2 / 0.7 = 4.571$$

In another embodiment, the computing system 100 may determine the grade score of the highest grade of highest likelihood value among the grades in the severity grading system as the effective grade for the determination target pixel p, and the computing system 100 may determine the grade score 5 of the grade G5 with the greatest likelihood of 0.4 as the effective grade in the case of FIG. 5B.

Next, the computing system 100 may determine the effective grade for the determination target partial image based on the effective grade of each pixel constituting the determination target partial image (S170).

In an embodiment, the computing system 100 may calculate the average value of the effective grade of each pixel constituting the determination target partial image and determine it as the effective grade for the determination target partial image.

However, the average value of the effective grade of the pixels is not necessarily determined as the effective grade of the determination target partial image, and this is only an example for ease of understanding. According to an embodiment, the computing system 100 may calculate a representative value of the effective grade of each pixel constituting the determination target partial image using various statistical techniques and determine it as the effective grade for the determination target partial image. The representative value of 2 or more values may be an average value of these values, but is not limited thereto, and may be calculated by any one or at least some of these values. For example, the representative value of 2 or more values may be a maximum value, a minimum value, a median value, an n-quartile value, an average value within an interquartile range (IQR), an average value of only values satisfying a predetermined condition (e.g., an average value of other values than a value exceeding a predetermined range), a histogram mode, and so on besides the average value.

The first deep learning model 121-1 shown in FIG. 5A is also a pre-trained artificial neural network. The training of the first deep learning model 121-1 shown in FIG. 5A is almost identical to the process shown in FIG. 4, except that the information tagged in the training image is information related to the lesion area or the information related to the severity grade of each pixel, which shall be omitted in detailed explanation.

Meanwhile, the computing system 100 may determine the slide level severity of the pathology slide image including partial images by aggregating the effective grades of the partial images determined by the patch level severity determination method as described above. FIG. 7 is a flowchart illustrating an example of a slide level severity determination method performed by the computing system 100.

Referring to FIG. 7, the computing system 100 may divide a determination target pathology slide image S in a predetermined unit size (S300). If the determination target pathology slide image S may be divided into N-ary partial images, the divided partial images may be $P_1$ through $P_N$. The unit size may be the same as the size of the input data inputted to the first deep learning model 121, and the size may be a pixel $\times$ b pixel (a and b each is an integer greater than or equal to 1).

For each of the plurality of determination target partial images $P_1$ through $P_N$, the computing system 100 may input each determination target partial image $P_i$ (the integer i ranges from 1 to N) into the first deep learning model 121 (S310, S320), and determine an effective grade $D_i$ for the determination target partial image $P_i$ based on a determination result of the determination target partial image $P_i$ outputted by the first deep learning model 121 receiving the determination target partial image $P_i$ (S330).

In step S330, the computing system 100 may use the patch level severity determination method described above in reference to FIG. 3 of FIG. 6 to determine the effective grade $D_i$ for the determination target partial image $P_i$.

Next, the computing system 100 may determine a slide level severity grade for the determination target pathology slide image S based on the effective grades $D_1$ through $D_N$ of the plurality of determination target partial images respectively (S340).

FIG. 8 is a diagram illustrating a specific example of applying a slide level severity determination method according to an embodiment of the present disclosure.

Referring to FIG. 8, the computing system 100 may obtain a determination target pathology slide image 10, and divide it into a plurality of partial images 20 having a predetermined unit size (S300). FIG. 8 shows an example of dividing into 15 partial images 20-1 to 20-15.

Meanwhile, the computing system 100 may input each of the plurality of divided partial images 20-1 to 20-15 into the first deep learning model 121 (S310, S320), and determine an effective grade 30 for each of the plurality partial images 20-1 to 20-15 based on a determination result of each of the plurality of partial images 20-1 to 20-15 outputted by the first deep learning model 121 (S330).

Next, the computing system 100 may determine the slide level severity grade for the determination target pathology slide image 10 based on the effective grade 30 for each of the plurality of partial images 20-1 to 20-15 (S340).

In step S340 of FIG. 7, there may be various methods for determining the slide level severity grade.

In an embodiment, the computing system 100 may calculate a representative value of the effective grades of the plurality of determination target partial images constituting the determination target pathology slide image, and determine the calculated representative value as the slide level severity grade for the whole determination target pathology slide image.

For example, the computing system 100 may calculate a maximum value, a minimum value, a median value, an n-quartile value of the grades of the determination target partial images constituting the determination target pathology slide image, an average value within an IQR, an average value of only values satisfying a specific condition (e.g., an average value of other values than a value exceeding a specific range), a histogram mode, and so on as the representative value.

More specifically with the example of FIG. 8, if the computing system 100 uses the average value as the representative value of the effective grades of the plurality of determination target partial images, the computing system 100 may determine the average value 3.46 of the effective grades of the entire 15 partial images 20-1 to 20-15 as the slide level severity grade of the determination target pathology slide image 10, in the example of FIG. 8.

Alternatively, if the computing system 100 uses the maximum value as the representative value of the effective grades of the plurality of determination target partial images, the computing system 100 may determine the maximum value 4.2 of the effective grades of the 15 partial images 20-1 to 20-15 as the slide level severity grade of the determination target pathology slide image 10, in the example of FIG. 8.

Alternatively, if the computing system 100 uses the minimum value as the representative value of the effective grades of the plurality of determination target partial images, the computing system 100 may determine the minimum value 2.9 of the effective grades of the 15 partial images 20-1 to 20-15 as the slide level severity grade of the determination target pathology slide image 10, in the example of FIG. 8.

Alternatively, if the computing system 100 uses an average value of some higher effective grades of the effective grades of the plurality of determination target partial images as the representative value of the effective grades of the plurality of determination target partial images, the computing system 100 may determine an average value 4.1 (=(4.2 + 4.1 + 4) / 3 of top three effective grades of the 15 partial images 20-1 to 20-15 as the slide level severity grade of the determination target pathology slide image 10, in the example of FIG. 8.

In another embodiment, the computing system 100 may determine an average value of effective grades over a specific threshold among the effective grades of the plurality of determination target partial images as the slide level severity grade for the determination target whole pathology slide image. Alternatively, if the computing system 100 uses an average value of the effective grades over the specific threshold among the effective grades of the plurality of determination target partial images as the representative value of the effective grades of the plurality of determination target partial images, the computing system 100 may determine an average value 3.9 of the effective grade over the threshold 3.5 among the effective grades of the 15 partial images 20-1 to 20-15 as the slide level severity grade of the determination target pathology slide image 10, in the example of FIG. 8.

Besides, it is apparent that there may be various methods for determining the slide level severity grade in step S340 of FIG. 7.

In an embodiment, the computing system 100 may also determine the slide level severity grade by using the deep learning, and in this case, the second deep learning model 122 may be used.

The second deep learning model 122 may be an artificial neural network pre-trained to output an output value for determining the severity grade for the pathology slide image if a data set including effective grades for a plurality of partial images respectively divided into the unit size is inputted. For example, the second deep learning model 122 may be a neural network which receives a data set 30 including the effective grades of the plurality of partial images respectively shown in FIG. 8 and outputs an output value to determine the slide level severity 40 for the pathology slide image 10.

According to the present embodiment, in step S340, the computing system 100 may determine the slide level severity grade for the determination target pathology slide image S based on the output value outputted by the second deep learning model 122 which receives the data set including the effective grades $D_1$ through $D_N$ for the plurality of partial images respectively of the determination target pathology slide image S.

The specific process of determining the slide level severity grade for the determination target pathology slide image S based on the output value outputted by the second deep learning model 122 may be similar to the effective grade determination process shown in FIG. 3 or FIG. 6. For example, similar to the process shown in FIG. 3, the second deep learning model 122 may output a likelihood value for each grade in the severity grading system, and the computing system 100 may determine as the slide level severity grade, a weighted average of the grade scores per grade in the severity grading system weighted by the likelihood value of each grade in the severity grading system, a weighted average of the grade scores for the plurality of higher likelihood values weighted by the likelihood value of each of the plurality of higher likelihood values of high likelihood values in the severity grading system, a grade score of the highest grade of the highest likelihood value among the grades of the severity grading.

The second deep learning model 122 may be a neural network pre-trained before being used to determine the slide level severity grade, and FIG. 9 is a diagram illustrating a process of training the second deep learning model 122.

Referring to FIG. 9, the computing system 100 may obtain a pathology slide image R for training (or a training pathology slide image), and divide the pathology slide image R for training into the unit size (S400). If the pathology slide image R for training is divided into N-ary partial images, the divided partial images for data set generation are $Q_1$ through $Q_N$.

Next, for each of the partial images for data set generation (or the data set generation partial images) $Q_i$ through $Q_N$, the computing system 100 may input the partial image $Q_i$ (an integer i is 1 through N) for data set generation into the first deep learning model 121 (S410, S420), and determine an effective grade $E_i$ for the partial image $Q_i$ for data set generation based on a determination result outputted by the first deep learning model 121 receiving the partial image $Q_i$ for data set generation (S430).

Next, the computing system 100 may train the second deep learning model 122 by inputting the data set including effective grades $E_1$ through $E_N$ for the partial images respectively for generating the plurality of data sets into the second deep learning model 122.

Meanwhile, the method according to an embodiment of the present disclosure may be implemented in the form of computer-readable program instructions and stored in a non-transitory recording medium, and a control program and a target program according to an embodiment of the present disclosure may also be stored in a non-transitory computer-readable recording medium. The non-transitory computer-readable recording medium includes all types of recording devices storing data which may be read by a computer system.

Program instructions recorded on the recording medium may be those specifically designed and configured for the present disclosure, or may be known and available to those skilled in the software field.

Examples of the non-transitory computer-readable recording medium include magnetic media such as a hard disk, a floppy disk, and a magnetic tape, optical media such as a compact disk (CD)-ROM and a digital versatile disk (DVD), magneto-optical media such as a floptical disk, and hardware devices specially configured to store and perform program instructions such as a ROM, a RAM, and a flash memory. In addition, the non-transitory computer-readable recording medium may be distributed in computer systems connected through a network, and computer-readable codes may be stored and executed in a distributed manner.

Examples of the program instructions include not only machine language code such as those produced by a compiler, but also high-level language code which may be executed by a device for electronically processing information using an interpreter, for example, a computer.

The hardware devices described above may be configured to operate as one or more software modules to perform the operations of the present disclosure, and vice versa.

The above description of the present disclosure is for illustrative purposes, and those skilled in the art to which the present disclosure pertains will understand that the present disclosure may be easily modified into other specific forms without changing the technical idea or essential features of the present disclosure. Therefore, the embodiments described above should be understood in all respects as illustrative and not restrictive. For example, each component described as unitary may be implemented in a distributed manner, and similarly, components described as distributed may also be implemented in a combined form.

The scope of the present disclosure is indicated by the appended claims rather than the detailed description above, and all changes or modified forms derived from the meaning and scope of the claims and their equivalent concepts should be construed as being included in the scope of the present disclosure.

Industrial Availability

The present disclosure may be used in a patch level severity determination method, a slide level severity determination method, and a computing system for performing the same.

## Claims

1. A slide level severity determination method performed in a computing system comprising a first deep learning model which is an artificial neural network pre-trained, if each of partial images obtained by dividing a pathology slide image into a predetermined unit size is inputted, to output a determination result for the inputted partial image, the slide level severity determination method comprising:

for each of a plurality of determination target partial images obtained by dividing a predetermined determination target pathology slide image into the unit size, determining an effect grade for the determination target partial image based on a determination result for the determination target partial image outputted by the first deep learning model receiving the determination target partial image; and
determining a slide level severity grade for the whole determination target pathology slide image based on the effective grade for each of the plurality of determination target partial images constituting the determination target pathology slide image,
wherein the determination result for the partial image outputted by the first deep learning model is a likelihood value for each grade in a predetermined histological severity grading system with respect to a predetermined disease, and a predetermined grade score is pre-assigned to each grade in the severity grading system, and
wherein the determining of the effective grade for the determination target partial image based on the determination result for the determination target partial image outputted by the first deep learning model receiving the determination target partial image comprises,
determining the effective grade for the determination target partial image based on the likelihood value of each grade in the severity grading system of the determination target partial image and the grade score for each grade in the severity grading system.

2. The slide level severity determination method of claim 1, wherein the determining of the effective grade for the determination target partial image based on the likelihood value of each grade in the severity grading system of the determination target partial image and the grade score for each grade in the severity grading system comprises:

determining the effective grade for the determination target pathology image by calculating a weighted average of grade scores for each grade in the severity grading system weighted by the likelihood value of each grade in the severity grading system;
determining the effective grade for the determination target pathology image by calculating a weighted average of grade scores for each of of a plurality of higher grades weighted by a likelihood value of each of the plurality of higher grades of high likelihood values among grades in the severity grading system; or
determining a grade score of the highest grade with the highest likelihood value among grades in the severity grading system as the effective grade for the determination target pathology image.

3. A slide level severity determination method performed in a computing system comprising a first deep learning model which is an artificial neural network pre-trained, if each of partial images obtained by dividing a pathology slide image into a predetermined unit size is inputted, to output a determination result for the inputted partial image, the slide level severity determination method comprising:

for each of a plurality of determination target partial images obtained by dividing a predetermined determination target pathology slide image into the unit size, determining an effect grade for the determination target partial image based on a determination result for the determination target partial image outputted by the first deep learning model receiving the determination target partial image; and
determining a slide level severity grade for the whole determination target pathology slide image based on the effective grade for each of the plurality of determination target partial images constituting the determination target pathology slide image,
wherein the determination result for the partial image outputted by the first deep learning model comprises a determination result for each pixel constituting the partial image, the determination result for each pixel is a likelihood value for each grade in a predetermined histological severity grading system with respect to a predetermined disease, and a predetermined grade score is pre-assigned to each grade in the severity grading system, and
the determining of the effective grade for the determination target partial image based on the determination result for the determination target partial image outputted by the first deep learning model receiving the determination target partial image comprises:

for each determination target pixel constituting the determination target partial image, determining the effective grade for the determination target pixel based on the likelihood value of each grade in the severity grading system of the determination target pixel and the grade score for each grade in the severity grading system; and

determining the effective grade for the determination target partial image based on the effective grade of each determination target pixel constituting the determination target partial image.

**4.** The slide level severity determination method of claim 3, wherein the determining of the effective grade for the determination target pixel based on the likelihood value of each grade in the severity grading system of the determination target pixel outputted by the first deep learning model and the grade score for each grade in the severity grading system comprises:

determining the effective grade of the determination target pixel by calculating a weighted average of grade scores for each grade in the severity grading system weighted by the likelihood value of each grade in the severity grading system of the determination target pixel;
determining the effective grade of the determination target pixel by calculating a weighted average of grade scores of a plurality of higher grades weighted by the likelihood value of each of the plurality of higher grades of high likelihood values among grades in the severity grading system of the determination target pixel; or
determining the grade score of the highest grade with the highest likelihood value among the grades in the severity grading system of the determination target pixel as the effective grade of the determination target pixel.

**5.** The slide level severity determination method of claim 3, wherein the determining of the effective grade for the determination target partial image based on the effective grade of each determination target pixel constituting the determination target partial image comprises,
determining the effective grade for the determination target pathology image by calculating a representative value of the effective grade of each determination target pixel constituting the determination target partial image.

**6.** The slide level severity determination method of claim 1 or claim 3, wherein the determining of the slide level severity grade for the whole determination target pathology slide image based on the effective grade for each of the plurality of determination target partial images constituting the determination target pathology slide image comprises,
determining the slide level severity grade for the whole determination target pathology slide image by calculating a representative value of the effective grade of each determination target partial image constituting the determination target pathology slide image.

**7.** The slide level severity determination method of claim 6, wherein the determining of the slide level severity grade for the whole determination target pathology slide image by calculating the representative value of the effective grade of each determination target partial image constituting the determination target pathology slide image comprises,
calculating as the representative value any one of a maximum value, a minimum value, an average value, a median value, and an average value within an interquartile range (IQR) of the effective grade of each determination target partial image constituting the determination target pathology slide image.

**8.** The slide level severity determination method of claim 1 or claim 3, wherein the computing system further comprises a second deep learning model which is an artificial neural network pre-trained to output an output value for determining the severity grade for the pathology slide image if a data set comprising the effective grade for each of the plurality of partial images obtained by dividing the pathology slide image into the unit size is inputted, and

wherein the determining of the severity grade for the determination target pathology slide image based on the effective grade for each of the plurality of determination target partial images constituting the determination target pathology slide image comprises,
determining the severity grade for the determination target pathology slide image based on the output value outputted by the second deep learning model receiving the data set comprising the effective grade for each of the plurality of determination target partial images constituting the determination target pathology slide image.

**9.** The slide level severity determination method of claim 8, wherein the second deep learning model is the artificial neural network pre-trained by a second deep learning model training method,
wherein the second deep learning model training method comprises:

obtaining a plurality of training pathology slide images;
for each of a plurality of training pathology slides, generating a data set corresponding to the training pathology slide image; and
training the second deep learning model by inputting each data set corresponding to the plurality of training pathology slide images into the second deep learning model, and

wherein the generating of the data set corresponding to the training pathology slide image comprises:

for each of a plurality of data set generation partial images obtained by dividing the training pathology slide image into the unit size, determining an effective grade for the data set generation partial image based on a determination result outputted by the first deep learning model receiving the data set generation partial image; and
generating the data set comprising the effective grade for each of the plurality of data set generation partial images.

**10.** A patch level severity determination method performed in a computing system comprising a first deep learning model which is an artificial neural network pre-trained, if a partial image obtained by dividing a pathology slide image into a predetermined unit size is inputted, to output a determination result for the inputted partial image, the patch level severity determination method comprising:

inputting a predetermined determination target partial image into the first deep learning model, and obtaining a determination result for the determination target partial image outputted by the first deep learning model, wherein the determination result for the determination target partial image is a likelihood value for each grade in a predetermined severity grading system for a predetermined disease, and a predetermined grade score is pre-assigned to each grade in the severity grading system; and
determining an effective grade for the determination target partial image based on the likelihood value of each grade in the severity grading system outputted by the first deep learning model receiving the determination target partial image and a grade score for each grade in the severity grading system.

**11.** A patch level severity determination method performed in a computing system comprising a first deep learning model which is an artificial neural network pre-trained, if a partial image obtained by dividing a pathology slide image into a predetermined unit size is inputted, to output a determination result for the inputted partial image, the patch level severity determination method comprising:

inputting a predetermined determination target partial image into the first deep learning model, and obtaining a determination result for the determination target partial image outputted by the first deep learning model, wherein the determination result outputted by the first deep learning model for the determination target partial image comprises a determination result for each pixel constituting the determination target partial image, the determination result for each pixel is a likelihood value for each grade in a predetermined severity grading system for a predetermined disease, and a predetermined grade score is pre-assigned to each grade in the severity grading system;
for each determination target pixel constituting the determination target partial image,
determining an effective grade for the determination target pixel based on the likelihood value of each grade in the severity grading system of the determination target pixel and a grade score for each grade in the severity grading system; and
determining an effective grade for the determination target pathology image based on the effective grade of each determination target pixel constituting the determination target partial image.

**12.** A computer program installed on a data processing device and recorded on a non-transitory medium for performing the method of any one of claims 1, 3, 10 and 11.

**13.** A non-transitory computer-readable recording medium on which a computer program to perform the method of any one of claims 1, 3, 10 and 11 is recorded.

**14.** A computing system comprising:

a processor; and
a memory,
wherein the memory stores a computer program and a first deep learning model which is an artificial neural network pre-trained, if each of partial images obtained by dividing a pathology slide image into a predetermined unit size is inputted, to output a determination result for the inputted partial image,
the computer program, when executed by the processor, controls the computing system to perform a slide level severity determination method,
wherein the slide level severity determination method comprises:

for each of a plurality of determination target partial images obtained by dividing a predetermined determination target pathology slide image into the unit size, determining an effect grade for the determination target partial image based on a determination result for the determination target partial image outputted by the first deep learning model receiving the determination target partial image; and

determining a slide level severity grade for the whole determination target pathology slide image based on the effective grade for each of the plurality of determination target partial images constituting the determination target pathology slide image,

wherein the determination result for the partial image outputted by the first deep learning model is a likelihood value for each grade in a predetermined histological severity grading system with respect to a predetermined disease, and a predetermined grade score is pre-assigned to each grade in the severity grading system, and

wherein the determining of the effective grade for the determination target partial image based on the determination result for the determination target partial image outputted by the first deep learning model receiving the determination target partial image comprises,

determining the effective grade for the determination target partial image based on the likelihood value of each grade in the severity grading system of the determination target partial image and the grade score for each grade in the severity grading system.

**15.** The computing system of claim 14, wherein the determining of the effective grade for the determination target partial image based on the likelihood value of each grade in the severity grading system outputted by the first deep learning model receiving the determination target partial image and the grade score for each grade in the severity grading system comprises:

determining the effective grade for the determination target pathology image by calculating a weighted average of the grade score for each grade in the severity grading system weighted by the likelihood value of each grade in the severity grading system;

determining the effective grade for the determination target pathology image by calculating a weighted average of grade scores of for a plurality of higher grades weighted by the likelihood value of each of the higher grades of high likelihood values among grades in the severity grading system; or

determining a grade score of the highest grade with the highest likelihood value among the grades in the severity grading system as the effective grade for the determination target pathology image.

**16.** A computing system comprising:

a processor; and

a memory,

wherein the memory stores a computer program and a first deep learning model which is an artificial neural network pre-trained, if each of partial images obtained by dividing a pathology slide image into a predetermined unit size is inputted, to output a determination result for the inputted partial image,

the computer program, when executed by the processor, controls the computing system to perform a slide level severity determination method,

wherein the slide level severity determination method comprises:

for each of a plurality of determination target partial images obtained by dividing a predetermined determination target pathology slide image into the unit size, determining an effect grade for the determination target partial image based on a determination result for the determination target partial image outputted by the first deep learning model receiving the determination target partial image; and

determining a slide level severity grade for the whole determination target pathology slide image based on the effective grade for each of the plurality of determination target partial images constituting the determination target pathology slide image,

wherein the determination result for the partial image outputted by the first deep learning model comprises a determination result for each pixel constituting the partial image, the determination result for each pixel is a likelihood value for each grade in a predetermined histological severity grading system with respect to a predetermined disease, and a predetermined grade score is pre-assigned to each grade in the severity grading system, and

wherein the determining of the effective grade for the determination target partial image based on the determination result for the determination target partial image outputted by the first deep learning model receiving the determination target partial image comprises:

for each determination target pixel constituting the determination target partial image, determining an effective grade for the determination target pixel based on the likelihood value of each grade in the severity grading system of the determination target pixel and the grade score for each grade in the severity grading system; and
determining the effective grade for the determination target partial image based on the effective grade of each determination target pixel constituting the determination target partial image.

**17.** The computing system of claim 16, wherein the determining of the effective grade for the determination target pixel based on the likelihood value of each grade in the severity grading system of the determination target pixel and the grade score for each grade in the severity grading system comprises:

determining the effective grade of the determination target pixel by calculating a weighted average of the grade score for each grade in the severity grading system weighted by the likelihood value of each grade in the severity grading system of the determination target pixel;
determining the effective grade of the determination target pixel by calculating a weighted average of grade scores of a plurality of higher grades weighted by the likelihood value of each of the plurality of higher grades with high likelihood values among grades in the severity grading system of the determination target pixel; or
determining the grade score of the highest grade with the highest likelihood value among the grades in the severity grading system of the determination target pixel as the effective grade of the determination target pixel.

**18.** The computing system of claim 16, wherein the determining of the effective grade for the determination target partial image based on the effective grade of each determination target pixel constituting the determination target partial image comprises,
determining the effective grade for the determination target pathology image by calculating a representative value of the effective grade of each determination target pixel constituting the determination target partial image.

**19.** The computing system of claim 14 or 16, wherein the determining of the slide level severity grade for the whole determination target pathology slide image based on the effective grade for each of the plurality of determination target partial images constituting the determination target pathology slide image comprises,
determining the slide level severity grade for the whole determination target pathology slide image by calculating a representative value of the effective grade of each determination target partial image constituting the determination target pathology slide image.

**20.** The computing system of claim 19, wherein the determining of the slide level severity grade for the whole determination target pathology slide image by calculating the representative value of the effective grade of each determination target partial image constituting the determination target pathology slide image comprises,
calculating as the representative value any one of a maximum value, a minimum value, an average value, a median value, and an average value within an interquartile range (IQR) of the effective grade of each determination target partial image constituting the determination target pathology slide image.

**21.** The computing system of claim 14 or 16, wherein the memory further stores a second deep learning model which is an artificial neural network pre-trained to output an output value for determining a severity grade for the pathology slide image if a data set comprising the effective grade for each of the plurality of partial images obtained by dividing the pathology slide image into the unit size is inputted, and

the determining of the severity grade for the determination target pathology slide image based on the effective grade for each of the plurality of determination target partial images constituting the determination target pathology slide image comprises,
determining the severity grade for the determination target pathology slide image based on the output value outputted by the second deep learning model receiving the data set comprising the effective grade for each of the plurality of determination target partial images constituting the determination target pathology slide image.

**22.** The computing system of claim 21, wherein the second deep learning model is

the artificial neural network pre-trained by a second deep learning model training method,
wherein the second deep learning model training method comprises:

obtaining a plurality of training pathology slide images;

for each of a plurality of training pathology slides, generating a data set corresponding to the training pathology slide image; and

training the second deep learning model by inputting data sets corresponding to the plurality of training pathology slide images respectively into the second deep learning model, and

wherein the generating of the data set corresponding to the training pathology slide image comprises:

for each of a plurality of data set generation partial images obtained by dividing the training pathology slide image into the unit size, determining an effective grade for the data set generation partial image based on a determination result outputted by the first deep learning model receiving the data set generation partial image; and

generating the data set comprising the effective grade for each of the plurality of data set generation partial images.

**23.** A computing system comprising:

a processor; and

a memory,

wherein the memory stores a computer program and a first deep learning model which is an artificial neural network pre-trained, if each partial image obtained by dividing a pathology slide image into a predetermined unit size is inputted, to output a determination result for the inputted partial image,

wherein the computer program, when executed by the processor, controls the computing system to perform a patch level severity determination method, and

wherein the patch level severity determination method comprises:

inputting a predetermined determination target partial image into the first deep learning model, and obtaining a determination result for the determination target partial image outputted by the first deep learning model, wherein the determination result for the determination target partial image is a likelihood value for each grade in a predetermined severity grading system for a predetermined disease, and a predetermined grade score is pre-assigned to each grade in the severity grading system; and

determining an effective grade for the determination target partial image based on the likelihood value of each grade in the severity grading system outputted by the first deep learning model receiving the determination target partial image and the grade score for each grade in the severity grading system.

**24.** A computing system comprising:

a processor; and

a memory,

wherein the memory stores a computer program and a first deep learning model which is an artificial neural network pre-trained, if each partial image obtained by dividing a pathology slide image into a predetermined unit size is inputted, to output a determination result for the inputted partial image,

wherein the computer program, when executed by the processor, controls the computing system to perform a patch level severity determination method, and

wherein the patch level severity determination method comprises:

inputting a predetermined determination target partial image into the first deep learning model, and obtaining a determination result for the determination target partial image outputted by the first deep learning model, wherein the determination result for the determination target partial image outputted by the first deep learning model comprises a determination result for each pixel constituting the determination target partial image, the determination result for each pixel is a likelihood value for each grade in a predetermined severity grading system for a predetermined disease, and a predetermined grade score is pre-assigned to each grade in the severity grading system;

for each determination target pixel constituting the determination target partial image,

determining an effective grade for the determination target pixel based on the likelihood value of each grade in the severity grading system of the determination target pixel and the grade score for each grade in the severity grading system; and

determining an effective grade for the determination target pathology image based on the effective grade of each determination target pixel constituting the determination target partial image.

FIG. 1

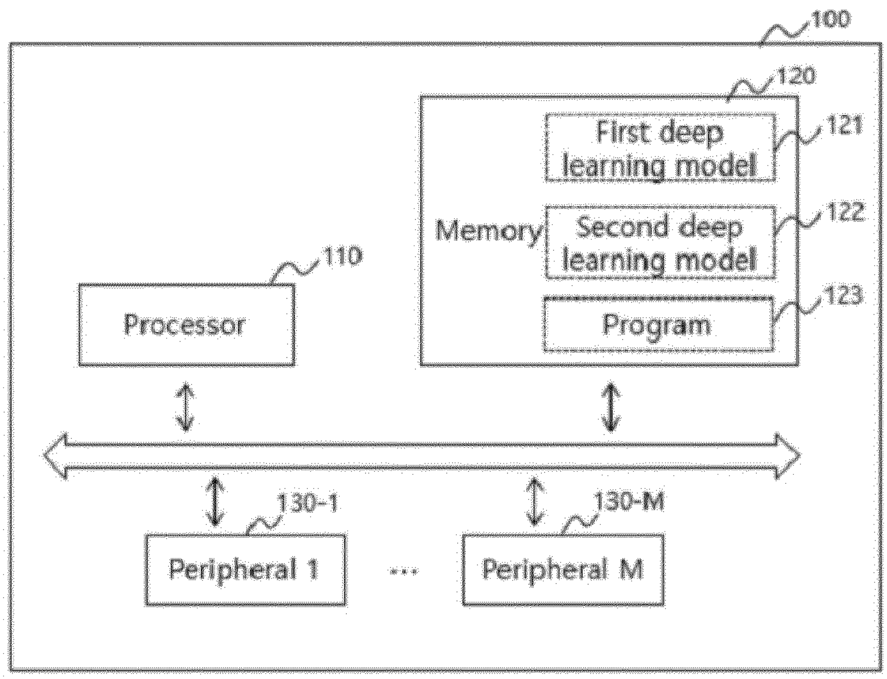
100
120
First deep learning model
121
Memory
Second deep learning model
122
110
Processor
Program
123
130-1
130-M
Peripheral 1
...
Peripheral M

FIG. 2

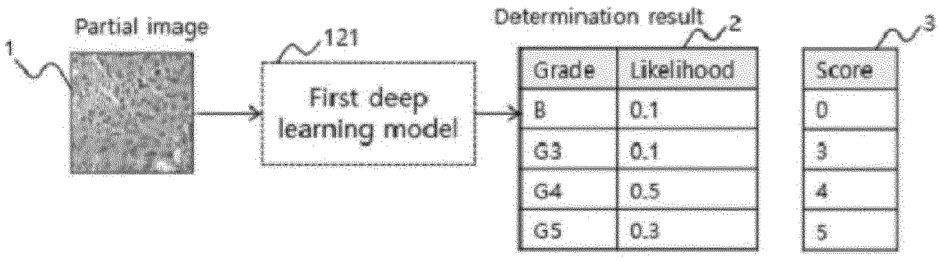
Partial image
1
121
First deep learning model
Determination result
2
Grade
Likelihood
B
0.1
G3
0.1
G4
0.5
G5
0.3
3
Score
0
3
4
5

FIG. 3

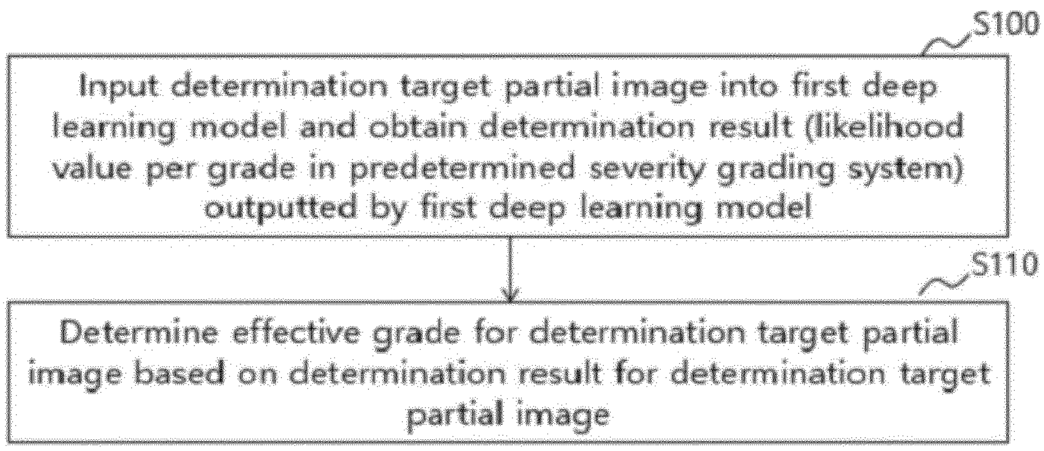
S100
Input determination target partial image into first deep learning model and obtain determination result (likelihood value per grade in predetermined severity grading system) outputted by first deep learning model
S110
Determine effective grade for determination target partial image based on determination result for determination target partial image

FIG. 4

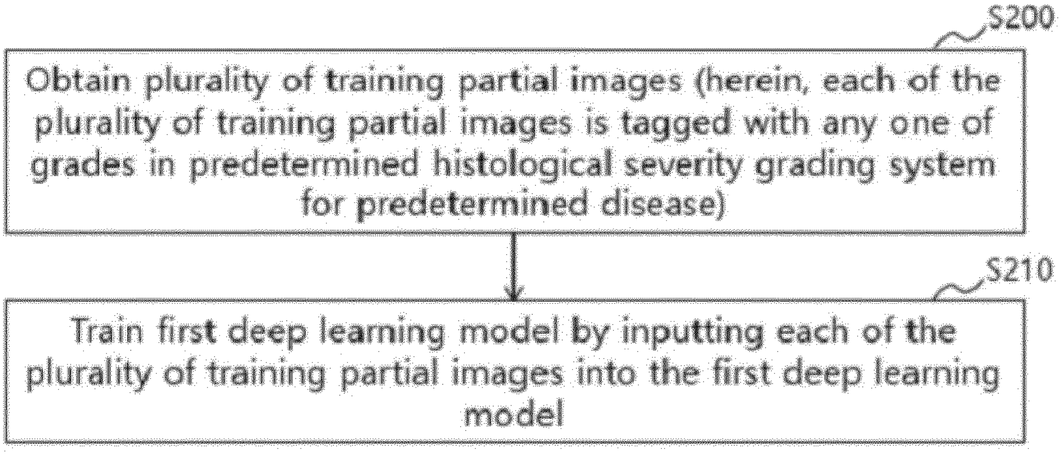
S200
Obtain plurality of training partial images (herein, each of the plurality of training partial images is tagged with any one of grades in predetermined histological severity grading system for predetermined disease)
S210
Train first deep learning model by inputting each of the plurality of training partial images into the first deep learning model

FIG. 5a

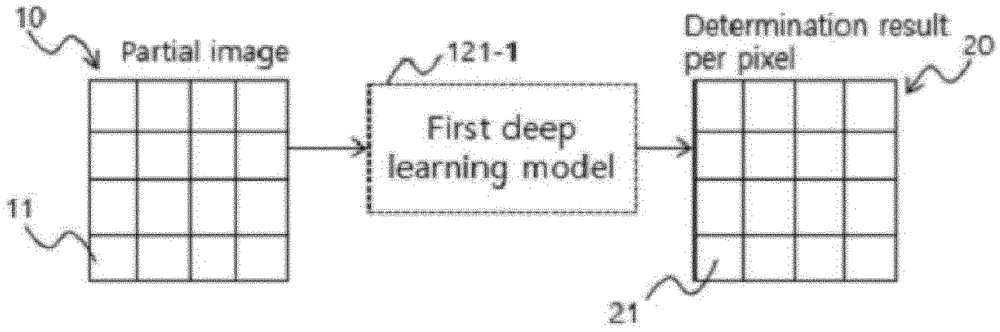


FIG. 5b

Determination result for pixel 11

21

| Grade | Likelihood |
|---|---|
| B | 0.2 |
| G3 | 0.1 |
| G4 | 0.3 |
| G5 | 0.4 |

3

| Score |
|---|
| 0 |
| 3 |
| 4 |
| 5 |

FIG. 6

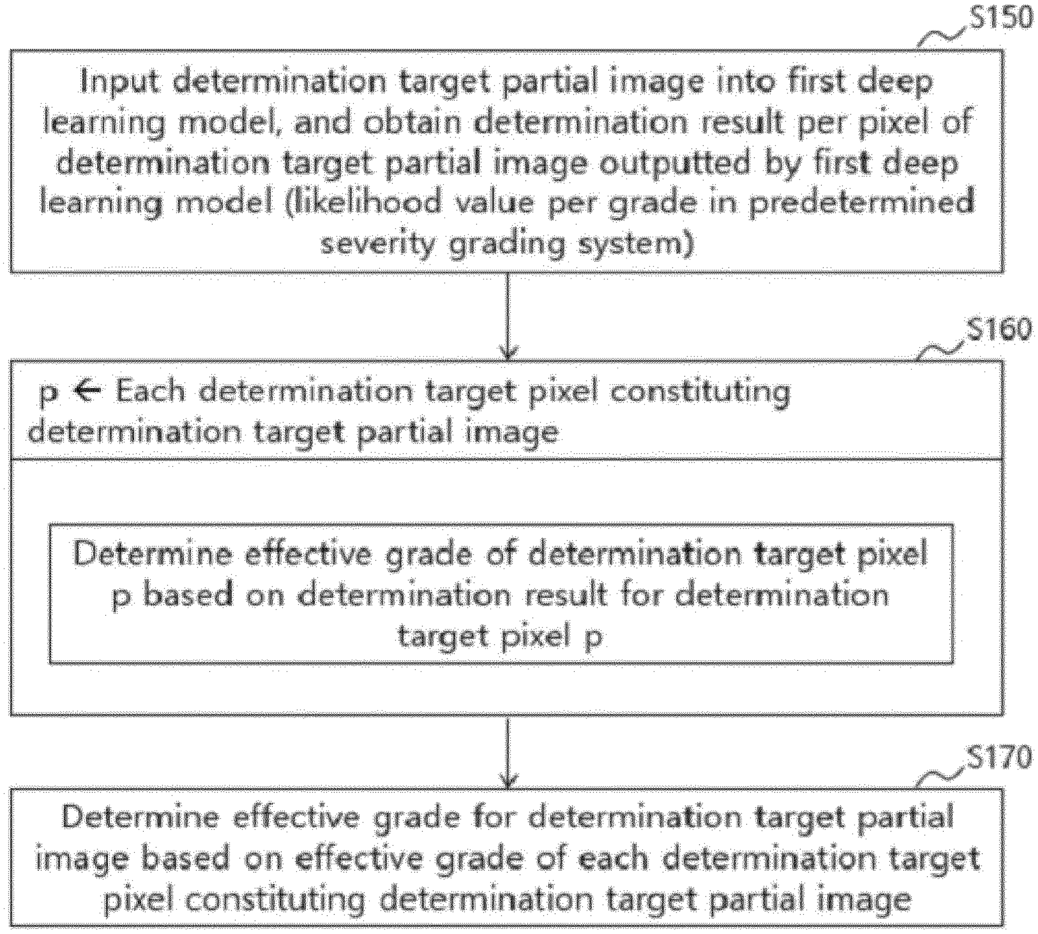
S150
Input determination target partial image into first deep learning model, and obtain determination result per pixel of determination target partial image outputted by first deep learning model (likelihood value per grade in predetermined severity grading system)
S160
p ← Each determination target pixel constituting determination target partial image
Determine effective grade of determination target pixel p based on determination result for determination target pixel p
S170
Determine effective grade for determination target partial image based on effective grade of each determination target pixel constituting determination target partial image

FIG. 7

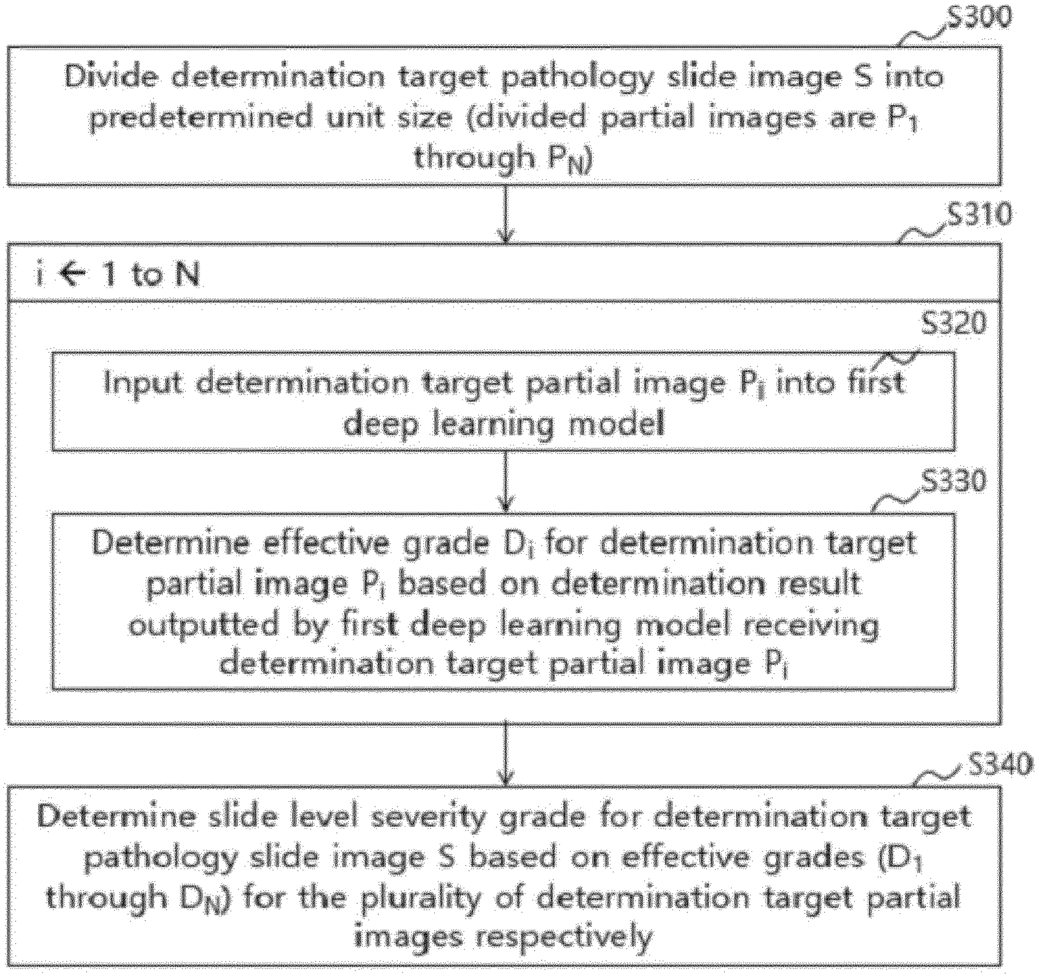
S300
Divide determination target pathology slide image S into predetermined unit size (divided partial images are $P_1$ through $P_N$)
S310
i ← 1 to N
S320
Input determination target partial image $P_i$ into first deep learning model
S330
Determine effective grade $D_i$ for determination target partial image $P_i$ based on determination result outputted by first deep learning model receiving determination target partial image $P_i$
S340
Determine slide level severity grade for determination target pathology slide image S based on effective grades ($D_1$ through $D_N$) for the plurality of determination target partial images respectively

FIG. 8

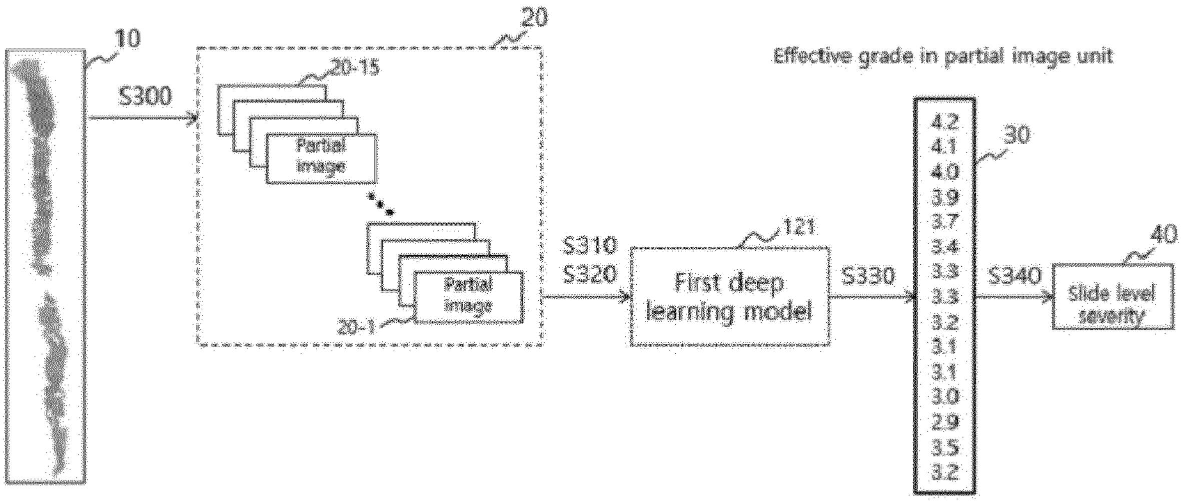
10
S300
20
20-15
Partial image
Partial image
20-1
S310
S320
121
First deep learning model
S330
Effective grade in partial image unit
4.2
4.1
4.0
3.9
3.7
3.4
3.3
3.3
3.2
3.1
3.1
3.0
2.9
3.5
3.2
30
S340
40
Slide level severity

FIG. 9

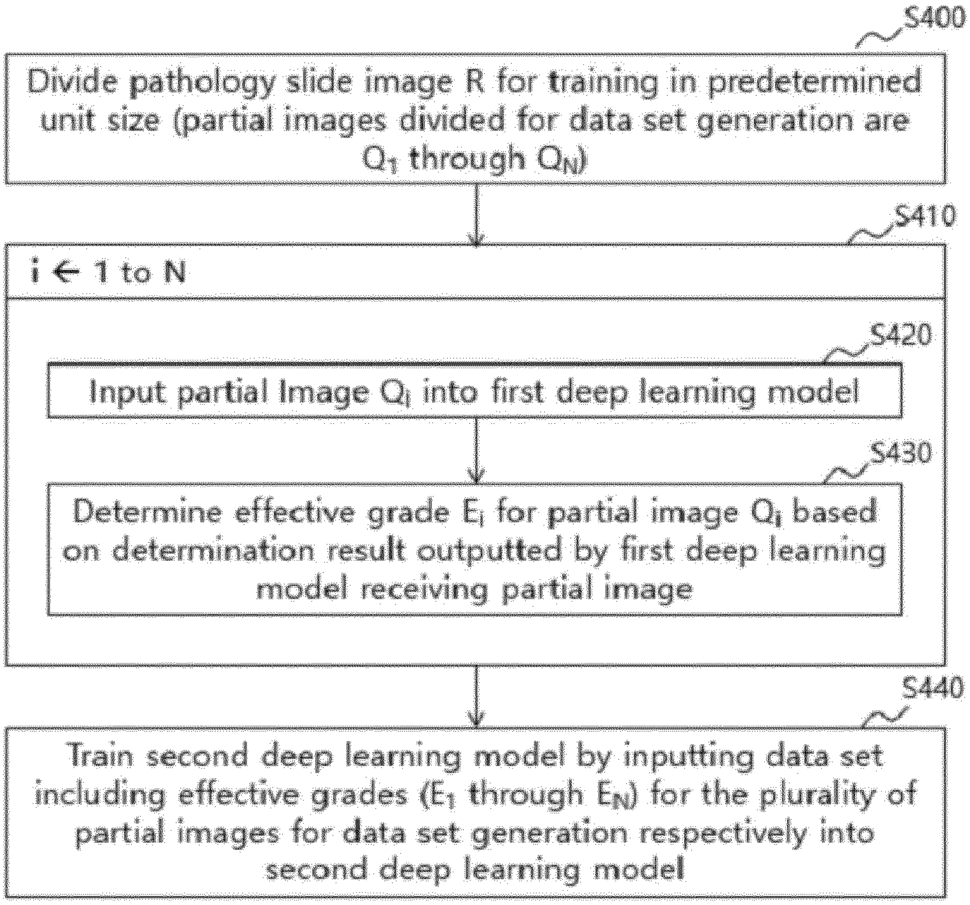
S400
Divide pathology slide image R for training in predetermined unit size (partial images divided for data set generation are $Q_1$ through $Q_N$)
S410
i ← 1 to N
S420
Input partial Image $Q_i$ into first deep learning model
S430
Determine effective grade $E_i$ for partial image $Q_i$ based on determination result outputted by first deep learning model receiving partial image
S440
Train second deep learning model by inputting data set including effective grades ($E_1$ through $E_N$) for the plurality of partial images for data set generation respectively into second deep learning model

## INTERNATIONAL SEARCH REPORT

International application No. **PCT/KR2023/007538**

**A. CLASSIFICATION OF SUBJECT MATTER**

**G16H 50/20**(2018.01)i; **G16H 50/30**(2018.01)i; **G16H 30/40**(2018.01)i; **G06N 3/04**(2006.01)i; **G06V 10/70**(2022.01)i; **G06V 20/69**(2022.01)i

According to International Patent Classification (IPC) or to both national classification and IPC

**B. FIELDS SEARCHED**

Minimum documentation searched (classification system followed by classification symbols)

G16H 50/20(2018.01); A61B 5/00(2006.01); G06N 3/04(2006.01); G06T 7/00(2006.01); G16H 70/60(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 슬라이드 이미지 (slide image), 인공 뉴럴 네트워크 (artificial neural network), 딥러닝 (deep learning), 등급 (grade), 결정 (determination)

**C. DOCUMENTS CONSIDERED TO BE RELEVANT**

| Category* | Citation of document, with indication, where appropriate, of the relevant passages | Relevant to claim No. |
|---|---|---|
| X | KR 10-2020-0016669 A (DEEP BIO INC.) 17 February 2020 (2020-02-17) See paragraphs [0075]-[0108]. | 10,12,13,23 |
| Y | | 11,24 |
| A | | 1-9,14-22 |
| Y | KR 10-2020-0092803 A (DEEP BIO) 04 August 2020 (2020-08-04) See paragraphs [0040]-[0048]. | 11,24 |
| A | KR 10-2261475 B1 (DEEP BIO INC.) 07 June 2021 (2021-06-07) See entire document. | 1-24 |
| A | JP 2022-527240 A (SPINTELLX, INC.) 01 June 2022 (2022-06-01) See entire document. | 1-24 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 October 2023** | **05 October 2023** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2023/007538

C. DOCUMENTS CONSIDERED TO BE RELEVANT

| Category* | Citation of document, with indication, where appropriate, of the relevant passages | Relevant to claim No. |
|---|---|---|
| PX | KR 10-2485415 B1 (DEEP BIO INC.) 06 January 2023 (2023-01-06)<br>See entire document.<br>This document is the document for the priority of the present international application. | 1-24 |

Form PCT/ISA/210 (second sheet) (July 2022)

**INTERNATIONAL SEARCH REPORT**
**Information on patent family members**

International application No.
**PCT/KR2023/007538**

| Patent document cited in search report | Publication date (day/month/year) | Patent family member(s) | Publication date (day/month/year) |
|---|---|---|---|
| KR 10-2020-0016669 A | 17 February 2020 | CN 112567474 A | 26 March 2021 |
| | | EP 3819910 A2 | 12 May 2021 |
| | | JP 2021-533471 A | 02 December 2021 |
| | | JP 7152810 B2 | 13 October 2022 |
| | | KR 10-2151723 B1 | 03 September 2020 |
| | | US 2021-0295509 A1 | 23 September 2021 |
| | | WO 2020-032561 A2 | 13 February 2020 |
| | | WO 2020-032561 A3 | 26 March 2020 |
| KR 10-2020-0092803 A | 04 August 2020 | KR 10-2236948 B1 | 06 April 2021 |
| KR 10-2261475 B1 | 07 June 2021 | CN 115699024 A | 03 February 2023 |
| | | EP 4148625 A1 | 15 March 2023 |
| | | JP 2023-529584 A | 11 July 2023 |
| | | US 2023-0229927 A1 | 20 July 2023 |
| | | WO 2021-246811 A1 | 09 December 2021 |
| JP 2022-527240 A | 01 June 2022 | CA 3133689 A1 | 24 September 2020 |
| | | CN 113892148 A | 04 January 2022 |
| | | EP 3938951 A1 | 19 January 2022 |
| | | MA 55302 A | 19 January 2022 |
| | | US 11367184 B2 | 21 June 2022 |
| | | US 2020-0294231 A1 | 17 September 2020 |
| | | US 2023-0142758 A1 | 11 May 2023 |
| | | WO 2020-190851 A1 | 24 September 2020 |
| KR 10-2485415 B1 | 06 January 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 102162895


### Non-patent literature cited in the description


- **NAGPAL, K.** ; **FOOTE, D.** ; **LIU, Y. et al.** Development and validation of a deep learning algorithm for improving Gleason scoring of prostate cancer. npj. *Digit. Med.*, 2019, vol. 2, 48, https://doi.org/10.1038/s41746-019-0112-2